(19) Europäisches Patentamt
European Patent Office
Office européen des brevets

(11) Numéro de publication: **0 601 080 B1**

# (12) FASCICULE DE BREVET EUROPEEN

(45) Date de publication de fascicule du brevet: **26.07.95**

(51) Int. Cl.$^6$: **A61K 7/42**

(21) Numéro de dépôt: **92919103.9**

(22) Date de dépôt: **25.08.92**

(86) Numéro de dépôt internationale :
**PCT/FR92/00820**

(87) Numéro de publication internationale :
**WO 93/04665 (18.03.93 93/08)**

## (54) COMPOSITION COSMETIQUE FILTRANTE CONTENANT UN POLYMERE FILTRE LIPOSOLUBLE A STRUCTURE HYDROCARBONEE ET UNE SILICONE FILTRE.

(30) Priorité: **29.08.91 FR 9110730**

(43) Date de publication de la demande:
**15.06.94 Bulletin 94/24**

(45) Mention de la délivrance du brevet:
**26.07.95 Bulletin 95/30**

(84) Etats contractants désignés:
**DE ES FR GB IT**

(56) Documents cités:
**EP-A- 0 392 882**
**FR-A- 2 601 365**

(73) Titulaire: **L'OREAL**
**14, rue Royale**
**F-75008 Paris (FR)**

(72) Inventeur: **FORESTIER, Serge**
**16, allée Ferdinand-Buisson**
**F-77410 Claye-Souilly (FR)**
Inventeur: **HANSENNE, Isabelle**
**156-158, rue Legendre**
**F-75017 Paris (FR)**

(74) Mandataire: **Casalonga, Axel et al**
**BUREAU D.A. CASALONGA - JOSSE**
**Morassistrasse 8**
**D-80469 München (DE)**

EP 0 601 080 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une composition cosmétique destinée à protéger les fibres kératiniques, et plus particulièrement la peau et les cheveux, contre les effets néfastes du rayonnement ultraviolet et contenant en association, un polymère hydrocarboné liposoluble filtrant les UV et un organopolysiloxane filtrant les UV.

On sait que les radiations lumineuses de longueurs d'onde comprises entre 280 nm et 400 nm permettent le brunissement de l'épiderme humain et que les rayons de longueurs d'onde comprises entre 280 et 320 nm, connues sous la dénomination d'UV-B, provoquent des érythèmes et des brûlures cutanées qui peuvent nuire au développement du bronzage ; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il est souhaitable que les compositions filtrant les UV présentent, outre un bon indice de protection dans l'UV-A et l'UV-B, de bonnes propriétés cosmétiques ainsi qu'une bonne rémanence à l'eau, c'est-à-dire une bonne stabilité de l'indice de protection au cours du temps, notamment après la douche ou la baignade.

L'indice de protection ou IP peut s'exprimer par le rapport du temps d'irradiation nécessaire pour atteindre le seuil érythématogène avec le filtre UV au temps nécessaire pour atteindre le seuil érythématogène sans filtre UV.

On a déjà proposé des compositions cosmétiques protectrices de l'épiderme humain, ou anti-solaires, renfermant des polymères filtres. On appellera "polymère filtre" un polymère hydrocarboné synthétique ou naturel porteur d'au moins un groupement absorbant les UV.

On a cependant constaté que ces compositions présentent des propriétés cosmétiques encore insuffisantes et forment sur la peau des films qui ne sont pas toujours souples et continus.

On a également déjà proposé l'utilisation d'organopolysiloxanes filtrant les UV ou "silicones filtres" dans des compositions cosmétiques protectrices de l'épiderme humain. On appellera "silicone filtre" une silicone renfermant au moins un groupement absorbant les UV. Ces compositions présentent de bonnes propriétés cosmétiques mais la demanderesse a constaté que leur rémanence à l'eau était généralement trop faible.

La demanderesse a découvert que l'association d'un polymère liposoluble et d'une silicone filtre permettait de remédier aux inconvénients des compositions de l'art antérieur.

L'association selon l'invention permet en effet de pallier aux insuffisances du polymère au niveau des propriétés cosmétiques et notamment au niveau de l'effet collant, de l'étalement et de la douceur sans toutefois, ce qui est surprenant, diminuer ses bonnes propriétés de rémanence.

Les compositions, selon l'invention, présentent donc des propriétés cosmétiques améliorées associées à une bonne rémanence à l'eau.

Il s'est avéré qu'en outre les compositions selon l'invention formaient un film protecteur continu et souple sur la peau.

L'invention a donc pour objet une composition cosmétique protectrice des fibres kératiniques et notamment de la peau et des cheveux, renfermant dans un support cosmétiquement acceptable, au moins un polymère filtre hydrocarboné liposoluble et au moins un organopolysiloxane filtrant les UV, le rapport en poids entre le polymère filtre et la silicone filtre étant compris entre O,1 et 5, et de préférence entre 0,2 et 2,5.

L'invention a également pour objet un procédé de protection de l'épiderme humain et des cheveux contre le rayonnement ultraviolet au moyen de ladite composition.

Les polymères filtres à chaîne hydrocarbonée liposolubles utilisés selon l'invention peuvent être

a) des polymères à structure polyéthylénique éventuellement substituée, de polyéthylèneimine, de chitine ou de chitosane, sur lesquels sont greffées des molécules absorbant le rayonnement ultraviolet par l'intermédiaire d'une fonction ester, amide, éther, thioéther, sulfonyle ou acyle,

b) des polymères résultant de l'homo- ou de la copolymérisation de molécules absorbant le rayonnement ultraviolet ("monomères filtres") portant un groupement insaturé choisi parmi les radicaux : allyle, vinyle, acrylamide, méthacrylamide, vinyloxycarbonylméthyle, acrylamidoalkyle et notamment acrylamidométhyle, méthacrylamidoalkyle, acrylamido(phényl)alkyle, méthacrylamido(phényl)alkyle, acryloxy,

acryloxyalkyle et acryloxypolyoxyéthylène, avec éventuellement d'autres monomères insaturés.

A titre de molécules absorbant le rayonnement ultraviolet, on peut citer les composés suivants :

- le benzylidène camphre et ses dérivés substitués sur le noyau benzénique
- l'isophtalylidène camphre et le téréphtalylidène camphre éventuellement substitués sur le noyau benzénique
- l'acide cinnamique éventuellement substitué par un ou plusieurs groupes alcoxy inférieurs et ses esters,
- l'acide salicylique et ses esters,
- l'acide benzoïque et ses esters,
- l'acide p-aminobenzoïque et ses dérivés alkylés sur le groupement amino et leurs esters
- les hydroxybenzophénones éventuellement substituées
- le dibenzoylméthane éventuellement substitué
- le benzotriazole et les 2-arylbenzotriazoles
- les 2 arylbenzimidazoles
- les 2-arylbenzofurannes
- les 2-arylbenzoxazoles
- les 2-arylindoles
- les mono- ou diphénylcyanoacrylates
- les absorbeurs de structure coumarinique.

A titre de monomères insaturés copolymérisables avec les "monomères filtres", on peut citer :

les acides acrylique, méthacrylique, itaconique, crotonique ou leurs esters, l'acrylamide et ses dérivés, le méthacrylamide et ses dérivés, l'acrylonitrile, le méthacrylonitrile, le styrène, l'α-méthylstyrène, l'isoprène, le butadiène, l'éthylène, le propylène, les esters vinyliques, les éthers vinyliques, les chlorures et fluorures de vinyle, le chlorure de vinylidène, la N-vinylpyrrolidone, la N-méthacryloyl D-glucosamine et les monoesters et diesters des acides maléique et fumarique.

Dans la présente invention, on entendra par "polymère liposoluble" un polymère insoluble dans l'eau à une concentration supérieure à 0,1% en poids à température ambiante et soluble ou dispersible dans une huile cosmétique telle que l'adipate d'isopropyle à une concentration d'au moins 1% en poids à température ambiante.

A titre de polymères filtres à chaîne hydrocarbonée liposolubles pouvant être utilisés selon l'invention, on peut citer à titre d'exemples :

1) les polymères comportant des motifs de formule (I)

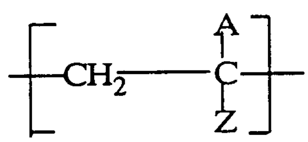

(I)

dans laquelle

Z désigne :

a) - $CONHCH_2X$, A désignant un atome d'hydrogène et X désignant un groupement aromatique absorbeur d'UV, tels que ceux décrits dans les brevets français n° 2 597 336, 2 237 912, 2 359 857 et 2 596 400, dans lesquels X est de préférence choisi parmi les radicaux benzylidène camphre, éventuellement substitués en positions 3 et 4 par un radical alcoxy en $C_1$-$C_{12}$ ou par un radical méthylènedioxy, les radicaux 2-(2'-hydroxyphényl)benzotriazole éventuellement substitués en position 5' par un radical méthyle ou tert.-octyle, le radical 4-méthoxy 4'-tert.-butyl-dibenzoylméthane, les radicaux 4-hydroxybenzophénone, 2-hydroxybenzophénone éventuellement substituée par un groupement méthoxy en position 4, les radicaux dérivés de 4-hydroxycoumarine et 7-hydroxycoumarine;

b)

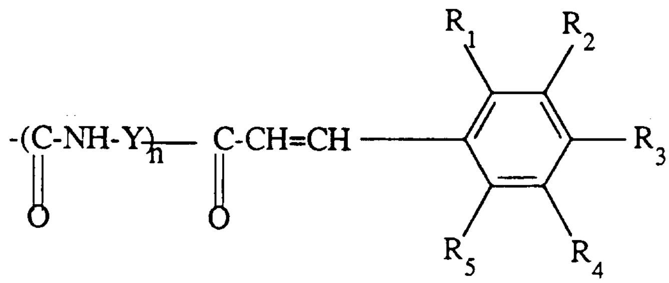

où n est 0 ou 1
lorsque n = 0, A représente un atome d'hydrogène;
lorsque n = 1, A représente un atome d'hydrogène ou un radical méthyle,
$R_1$, $R_2$, $R_3$, $R_4$, $R_5$, identiques ou différents,
représentent un atome d'hydrogène, un radical alkyle linéaire ou ramifié ayant de 1 à 8 atomes de carbone, un radical alcoxy ayant de 1 à 4 atomes de carbone, un radical dialkylamino ou dialkylaminoalkyle de formule :

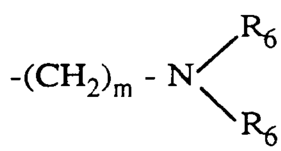

dans laquelle m est 1 à 3 et $R_6$ représente un radical méthyle ou éthyle, lesdits radicaux étant éventuellement quaternisés à l'aide d'un agent de quaternisation pris dans le groupe constitué par le chlorure de méthyle, le bromure de dodécyle, le sulfate de diméthyle et l'acide chloroacétique;
Y étant nul ou pouvant désigner $-CH_2-$, $-CH(CH_3)-$, $> CH-CH_2-CH(CH_3)_2$, $> CH-CH_2-C_6H_5$, $-C(CH_3)_2-CH_2-$, ces polymères étant décrits dans le brevet français n° 2 617 399;

2) les polymères dérivés d'acétate de vinyle comportant le motif de formule (II)

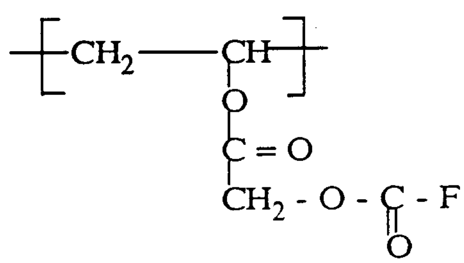
(II)

dans laquelle F représente un radical absorbeur d'UV, tels que ceux décrits dans les brevets français n° 2 197 023 et 2 359 856;
le groupement

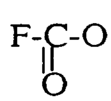

étant choisi de préférence parmi les groupes p-dialkyl($C_1$-$C_4$)aminobenzoate, cinnamate éventuellement substitué par un méthoxy, salicylate, diphénylcyanoacrylate et flufénate ou 3'-trifluorométhyldiphénylamine 2-carboxylate;

3) les copolymères de polyéthylèneimine de poids moléculaire compris entre 500 et 100 000 et de chlorure d'acide para dialkyl ($C_1$-$C_4$) aminobenzoïque tels que ceux décrits dans le brevet US 3 864 473;

4) les copolymères de monomères à insaturations éthyléniques et de dérivés d'acide 4-(N,N-diallylamino)benzoïque ou ses esters tels que ceux décrits dans le brevet US 3 795 733;

5) les produits de polymérisation des monomères de formule

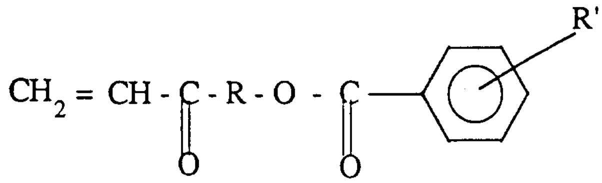

dans laquelle

R désigne

$$-(X)_n-O- \text{ ou } -(CH_2CH_2-O)_n- \quad ,$$

X désignant un radical divalent alkylène, arylène, alkylarylène,

n = 1 à 1000,

R' désigne OH, $N(R_1)_2$,

$R_1$ désigne H, alkyle, aryle, alkylaryle,

les radicaux alkyle ayant de 1 à 20 atomes de carbone,

tels que ceux décrits par exemple dans le brevet WO 88/09783;

6) les polymères dérivés de chitine et de chitosane portant au moins un groupement absorbeur d'UV de formule :

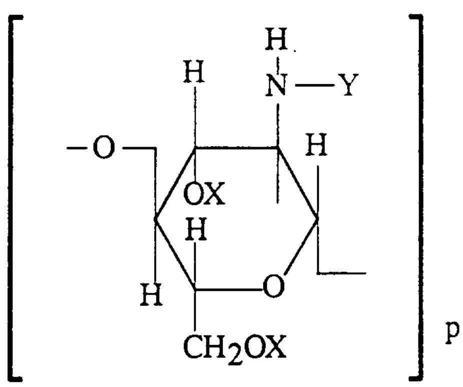

(III)

dans laquelle :

X et Y, indépendamment l'un de l'autre, désignent hydrogène, un radical benzoyle de formule (IV) ou un radical cinnamoyle de formule (V) et Y peut aussi désigner acétyle :

(IV) 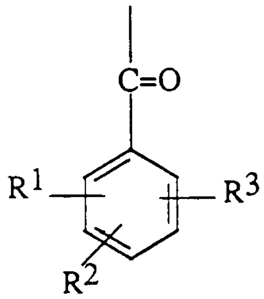

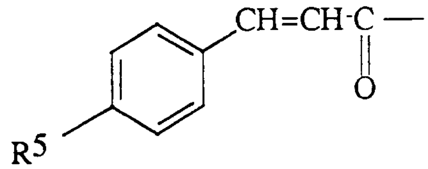 (V)

formules dans lesquelles :

$R^1$, $R^2$ et $R^3$, identiques ou différents, désignent hydrogène, alcoxy en $C_1$-$C_4$, hydroxy ou

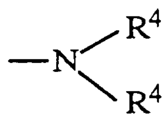

$R^4$ désignant hydrogène, alkyle en $C_1$-$C_4$, monohydroxyalkyle en $C_2$-$C_4$ ou dihydroxyalkyle en $C_2$-$C_4$ sous réserve qu'au moins un des groupements $R^1$, $R^2$ ou $R^3$ ne désigne pas hydrogène et qu'au plus un des groupements $R^1$, $R^2$ ou $R^3$ désigne

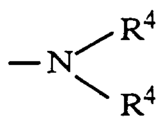

$R^5$ désigne alcoxy en $C_1$-$C_4$

p = 3 - 20 000,

sous réserve que lorsque X désigne hydrogène, Y ne désigne ni hydrogène, ni acétyle;

de tels polymères sont décrits dans la demande de brevet allemand n° 3 912 122.

Les polymères filtres liposolubles à chaîne hydrocarbonée préférés selon l'invention sont les polyacrylamides à greffons X benzylidènecamphre ou 2-(2'-hydroxyphényl)benzotriazole éventuellement substitués tels que définis ci-dessus en 1) a).

Les polymères filtres à chaîne siloxanique utilisés selon l'invention sont des diorganopolysiloxanes comportant dans leur molécule au moins une unité de formule :

$$X - \overset{R'_a}{\overset{|}{Si}} - O_{\frac{3-a}{2}} \quad (VI)$$

dans laquelle

R' désigne un groupe hydrocarboné saturé ou insaturé en $C_1$-$C_{30}$, un groupe hydrocarboné halogéné en $C_1$-$C_8$ ou un groupe triméthylsilyloxy;

a = 1 ou 2;

X = -A-Y

où A représente un radical divalent hydrocarboné aliphatique ou aromatique comportant au moins 2 atomes de carbone et renfermant éventuellement un ou plusieurs atomes d'oxygène;

Y représente le reste d'une molécule filtrant le rayonnement ultraviolet.

En plus des unités de formule (VI) le diorganopolysiloxane peut comporter des unités de formules :

$$R'_b - Si\, O_{\frac{4-b}{2}} \quad (VII) \quad \text{et} \quad Z - \overset{\displaystyle R'_a}{\overset{|}{Si}} - O_{\frac{3-a}{2}} \quad (VIII)$$

dans lesquelles R' et a ont la même signification que dans la formule (VI);

b est un nombre entier désignant 1, 2 ou 3;

Z = - O - Y, Y ayant la même signification que dans la formule (VI).

A titre de groupe hydrocarboné, ou peut citer les radicaux alkyle en $C_1$-$C_{30}$, alcényle en $C_2$-$C_{30}$, cycloalkyle ou aromatique comme phényle ou tolyle.

A titre de groupe hydrocarboné halogéné, on peut citer le radical 3,3,3,-trifluoropropyle.

Dans le diorganopolysiloxane constitué de motifs (VI) et éventuellement (VII) et (VIII), au moins 40% en nombre des radicaux R' sont des radicaux méthyle. Le nombre total des unités (VI), (VII) et (VIII) est de préférence inférieur ou égal à 250 et est compris en particulier entre 2 et 50.

Y représente de préférence un reste :

- benzylidène camphre éventuellement substitué sur le noyau benzénique par des radicaux hydroxyle, alkyle ou alcoxy en $C_1$-$C_6$;
- benzalmalonate de dialkyle en $C_1$-$C_8$, éventuellement substitué sur le noyau benzénique par des radicaux hydroxy, alkyle ou alcoxy en $C_1$-$C_6$;
- 2-(2'-hydroxyphényl)benzotriazole portant éventuellement sur l'un des noyaux aromatiques des substituants alkyle en $C_1$-$C_8$ ou alcényle en $C_2$-$C_8$, halogène, alcoxy, carboxy, hydroxy, amino ou tétraalkylpipéridyle;
- dibenzoylméthane portant éventuellement des substituants alkyle ou alcoxy en $C_1$-$C_8$ ou hydroxy;
- benzophénone portant éventuellement des substituants alkyle ou alcoxy en $C_1$-$C_8$ ou hydroxy;
- benzoate substitué par des radicaux hydroxy, alcoxy en $C_1$-$C_6$,amino ou mono ou di($C_1$-$C_6$ alkyl)-amino;
- cinnamate portant éventuellement des substituants hydroxy, alkyle ou alcoxy en $C_1$-$C_6$, amino ou mono ou di($C_1$-$C_6$) alkylamino;
- bis - ou tris (phénylacrylate) éventuellement substitué par des radicaux hydroxy ou alcoxy en $C_1$-$C_4$.

De tels polymères filtres à chaîne siloxanique sont décrits dans les demandes de brevet européen n° 0 335 777, 0 305 059, 0 392 882, 0 388 218, 0 392 883, 0 350 314, 0 383 655 et 0 389 337 et dans les demandes de brevet français 2 550 787 et 2 657 351 et dans les brevets américains n° 4 696 969, 4 554 369, 4 545 980, 4 562 278, 3 513 184 et 4 859 759.

Les silicones filtres préférées pour être utilisées selon l'invention sont celles dans lesquelles le radical Y désigne un reste benzylidène camphre, 2-(2'-hydroxyphényl)benzotriazole ou cinnamate éventuellement substitué tel que défini ci-dessus.

Les compositions protectrices de l'épiderme humain selon l'invention peuvent se présenter sous la forme d'émulsions, de lotions, de gels, d'huiles, de bâtonnets solides, de dispersions vésiculaires ou sous forme de compositions pour aérosol.

La concentration totale en polymère filtre et silicone filtre varie de 0,5 à 15% en poids par rapport au poids total de la composition.

Elles peuvent contenir les adjuvants cosmétiques habituellement utilisés dans ce type de compositions tels que des épaississants, des adoucissants, des humectants, des tensio-actifs, des conservateurs, des anti-mousses, des parfums, des huiles, des cires, des propulseurs, des colorants et/ou des pigments ayant pour fonction de colorer la composition elle-même ou la peau.

Comme solvant de solubilisation, on peut utiliser une huile, une cire et de façon générale tout corps gras, un monoalcool ou un polyol inférieur, un benzoate d'alcools en $C_{12}$-$C_{15}$ ou leurs mélanges. Les monoalcools ou polyols plus particulièrement préférés sont l'éthanol, l'isopropanol, le propylèneglycol, la glycérine et le sorbitol.

Une forme de réalisation de l'invention est une émulsion sous forme de crème ou de lait protecteurs comprenant en plus du polymère filtre et de la silicone filtre, des alcools gras, des esters d'acides gras, de la lanoline, des huiles ou cires naturelles ou synthétiques et des émulsionnants en présence d'eau.

Une autre forme de réalisation est constitué par des lotions huileuses à base d'huiles et cires naturelles ou synthétiques, de lanoline et d'esters d'acides gras, notamment de triglycérides d'acides gras, ou par des lotions oléoalcooliques à base d'un alcool inférieur tel que l'éthanol et d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylène glycol ou la glycérine et un épaississant tel que la silice. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

Dans le cas où la composition se présente sous forme de dispersion vésiculaire de lipides amphiphiles ioniques ou non ioniques, on la prépare selon des procédés connus, par exemple en faisant gonfler les lipides dans une solution aqueuse pour former des sphérules dispersées dans le milieu aqueux comme décrit dans l'article BANGHAM, STANDISH and WATKINS, J. Mol. Biol. 13, 238 (1965) ou dans les brevets FR 2 315 991 et 2 416 008.

Dans le cas d'une composition conditionnée en aérosol, on utilise les propulseurs classiques bien connus de l'état de la technique.

La présente invention vise également les compositions cosmétiques anti-solaires contenant au moins un polymère filtre et une silicone filtre et pouvant contenir d'autres filtres UV-B et/ou UV-A.

Dans ce cas, la quantité totale de filtres présents dans la composition anti-solaire est comprise entre 0,5 et 20% en poids par rapport au poids total de la composition anti-solaire.

Ces compositions anti-solaires se présentent sous les formes indiquées ci-dessus pour les compositions protectrices de l'épiderme humain.

Lorsque la composition cosmétique selon l'invention est destinée à protéger des rayons UV les cheveux naturels ou sensibilisés, cette composition peut se présenter sous forme de shampooing, de lotion, de gel ou émulsion à rincer, à appliquer avant ou après le shampooing, avant ou après coloration ou décoloration, avant ou après permanente ou défrisage, de lotion ou gel coiffants ou traitants, de lotion ou gel pour le brushing ou la mise en plis, de spray de coiffage, de laque pour cheveux, de composition de permanente ou de défrisage, de coloration ou décoloration des cheveux. Cette composition peut contenir divers adjuvants utilisés dans ce type de composition, tels que des agents tensio-actifs, des épaississants, des polymères, des adoucissants, des conservateurs, des stabilisateurs de mousse, des électrolytes, des solvants organiques, des huiles, des cires, des agents anti-gras, des colorants et/ou pigments ayant pour fonction de colorer la composition elle-même ou la chevelure ou tout autre ingrédient habituellement utilisé dans le domaine capillaire.

Dans ce cas la concentration totale en polymère filtre et en silicone filtre varie de 0,25 à 5% en poids par rapport au poids total de la composition capillaire.

Cette composition capillaire peut également contenir d'autres filtres UV-B et/ou UV-A.

La présente invention a aussi pour objet un procédé de protection de l'épiderme humain et des cheveux contre les rayons UV consistant à appliquer sur la peau ou les cheveux une quantité efficace d'une composition cosmétique telle que définie ci-dessus.

L'invention sera mieux illustrée par les exemples non limitatifs ci-après.

EXEMPLE 1

On prépare une émulsion antisolaire huile-dans-eau de composition suivante :

- Polyacrylamide à greffons benzylidènecamphre décrit dans l'exemple 1 du brevet français n° 2 597 336, constitué d'unités :

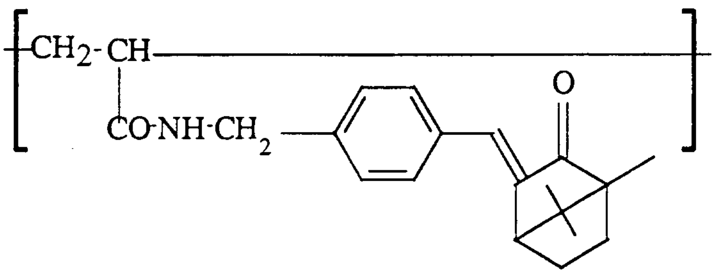

5 g

- Polydiméthylsiloxane à greffons 4'-triméthylèneoxy-3-benzylidènecamphre correspondant à la demande de brevet EP 0335 777, de formule : 5 g

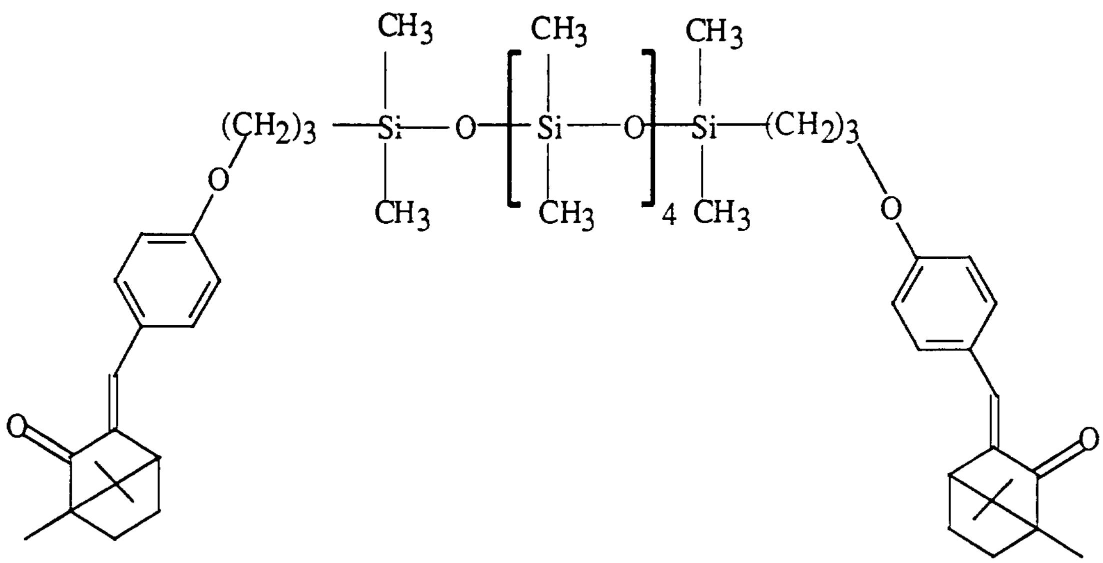

| | | |
|---|---|---|
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | | 7 g |
| - Mélange de mono et distéarate de glycérol non autoémulsionnable | | 2 g |
| - Benzoate d'alcools en $C_{12}$-$C_{15}$ vendu sous la dénomination "FINSOLV TN" par la Société WITCO | | 15 g |
| - Polydiméthylsiloxane | | 1,5 g |
| - Alcool cétylique | | 1,5 g |
| - Glycérine | | 20 g |
| - Conservateurs, parfum | qs | |
| - Eau | qsp | 100 g |

EXEMPLES 2 à 5

On prépare des émulsions en remplaçant dans l'émulsion de l'exemple 1 le polymère filtre et la silicone filtre par les composés suivants :

EXEMPLE 2

- Polyacrylamide à greffons 4-hexyloxybenzylidènecamphre dont la préparation est décrite dans l'exemple 5 du brevet français n° 2 596 400, constitué de motifs de formule : 5 g

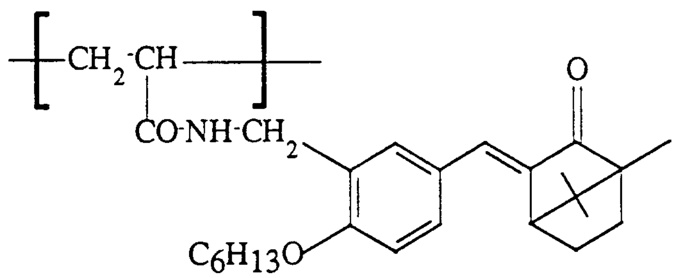

- Polydiméthylsiloxane à greffons 2-(3'-triméthylène-2'-hydroxy-5'-méthylphényl)benzotriazole de l'exemple 1 de la demande EP-A 0 392 883, de formule : 5 g

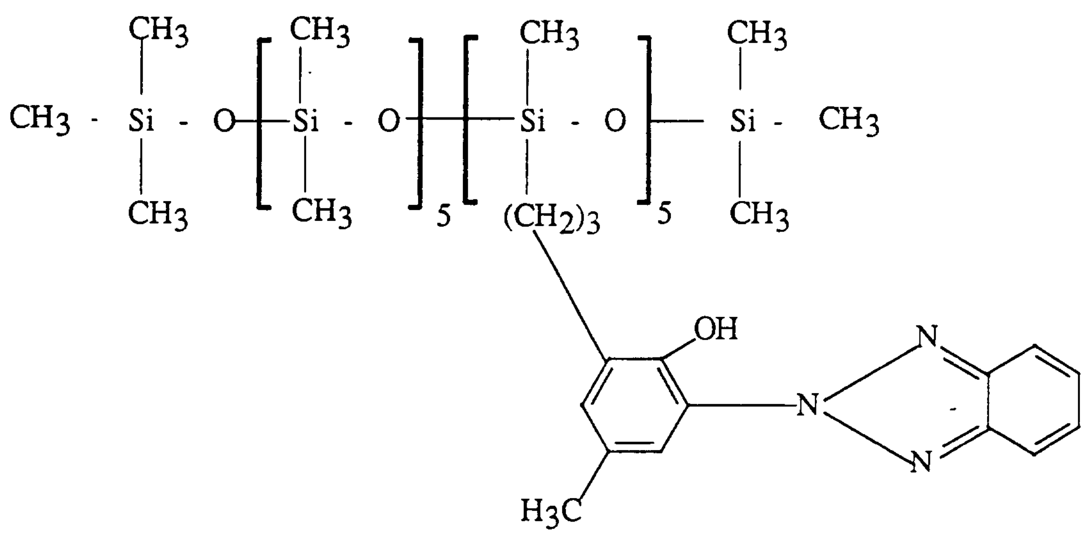

EXEMPLE 3

- Polymère filtre à greffons dérivés de benzotriazole comprenant 20% de motifs de formule :

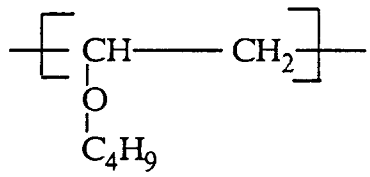

et 80% de motifs de formule :

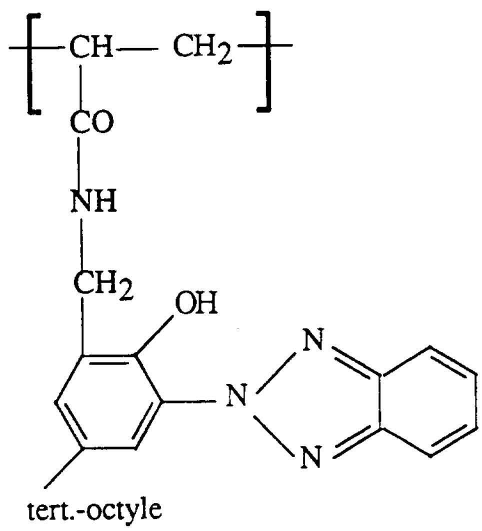

5 g

La masse moléculaire de ce polymère a été définie par chromatographie en phase gazeuse sur colonne Microstyragel, éluant tétrahydrofuranne, étalon polystyrène. Le sommet de la courbe de distribution des masses se situe à M = 8 000, 60% de la surface de la courbe étant comprise entre les masses 3 000 et 18 000.

- Polydiméthylsiloxane à greffons 2-méthyl 3-(4'-diméthylaminobenzoyloxy)propylène de formule : 5 g

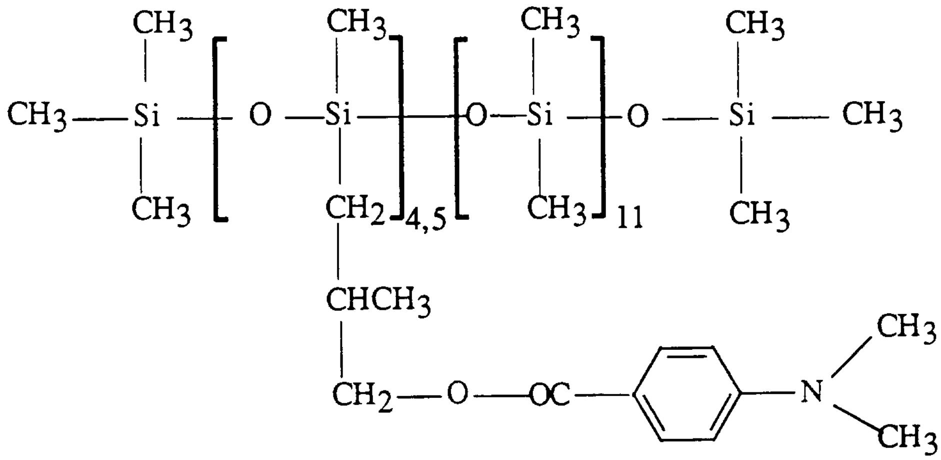

EXEMPLE 4

- Polyacrylamide filtre de l'exemple 1 5 g
- Mélange de polydiméthylsiloxanes à greffons 4'-triméthylèneoxy 3-benzylidènecamphre et 4'-oxy 3-benzylidènecamphre, préparé selon la demande EP 0335 777, ayant pour formule : 5 g

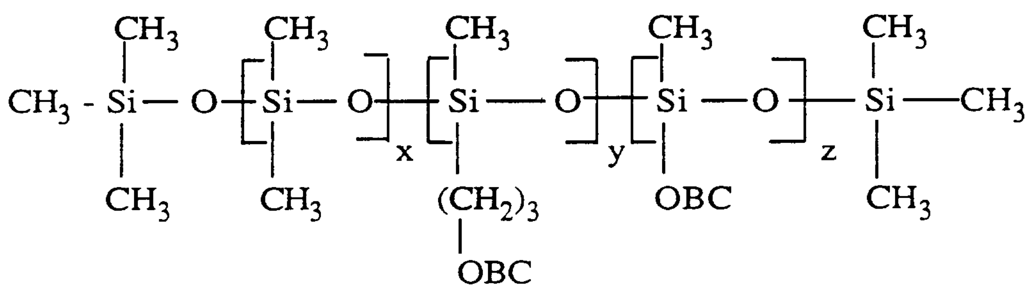

constitué d'environ :

- 55% de polydiméthylsiloxane dans lequel x = 0 à 20, y = 1 à 4, z = 0
- 5% de polydiméthylsiloxane dans lequel x = 0 à 21, y = 0, z = 1 à 4
- 40% de polydiméthylsiloxane dans lequel x = 0 à 16, y = 1 à 3, z = 1 à 3,

BC désignant le radical 3-benzylidènecamphre.

EXEMPLE 5

| | | |
|---|---|---|
| - Polymère filtre à greffons dérivés de benzotriazole de l'exemple 3 | 5 | g |
| - Polydiméthylsiloxane à greffons 2-(3'-triméthylène-2'-hydroxy-5'-méthylphényl)benzotriazole selon la demande EP 0392 883 | 5 | g |

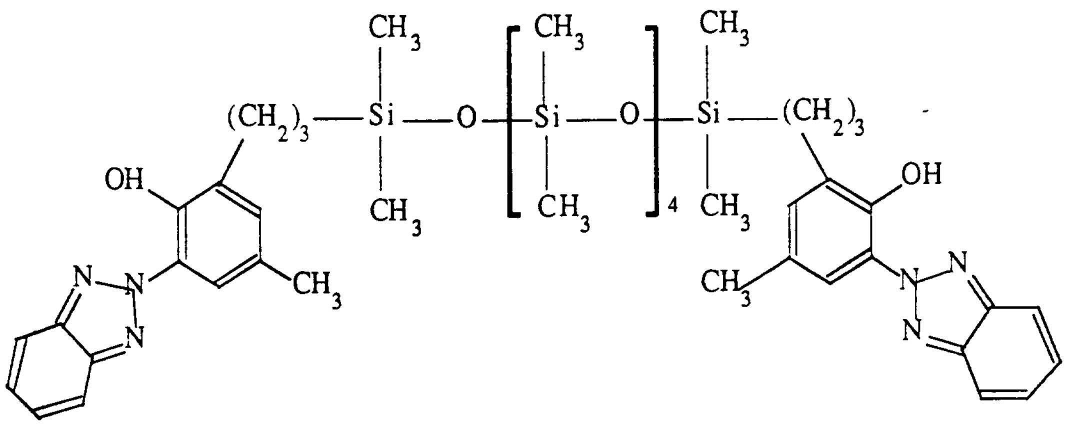

EXEMPLE 6

On prépare une huile solaire de composition suivante :

| | |
|---|---|
| - Polyacrylamide filtre de l'exemple 1 | 10 g |
| - Polydiméthylsiloxane filtre de l'exemple 1 | 4 g |
| - Ester palmitique du 2-éthylhexyl glycérol éther | 36 g |
| - Conservateurs qs | |
| - Adipate de diisopropyle qsp | 100 g |

EXEMPLE 7

On prépare une huile solaire de composition suivante :

| | |
|---|---|
| - Polyacrylamide filtre de l'exemple 1 | 10 g |
| - Silicone filtre de l'exemple 4 | 4 g |
| - Ester palmitique du 2-éthylhexylglycéroléther | 36 g |
| - Conservateurs qs | |
| - Adipate de diisopropyle qsp | 100 g |

EXEMPLE 8

On prépare un soin protecteur capillaire à rincer de composition suivante :

| | |
|---|---|
| - Polyacrylamide filtre de l'exemple 1 | 1 g |
| - Silicone filtre de l'exemple 4 | 0,7 g |
| - Mélange d'alcool cétylstéarylique et d'alcool cétylstéarylique oxyéthyléné à 33 moles d'oxyde d'éthylène | 2 g |
| - Monoéthanolamide de coprah | 0,5 g |
| - Gomme de xanthane vendue sous la dénomination "KELTROL T" par la Société KELCO | 0,8 g |
| - Conservateurs, parfum qs | |
| - HCl qs pH = 6,5 | |
| - Eau qsp | 100 g |

EXEMPLE 9

On prépare une crème solaire sous forme d'émulsion E/H de composition suivante :

| | | |
|---|---|---|
| - Polyacrylamide filtre de l'exemple 1 | | 3 g |
| - Silicone filtre de l'exemple 4 | | 6 g |
| - 2-octyl dodécanol-1 | | 10 g |
| - Stéarate de magnésium | | 4 g |
| - Lanoline hydrogénée | | 1 g |
| - Lanoline | | 4 g |
| - Cire d'abeilles | | 5 g |
| - Sesquioléate de sorbitan vendu sous la dénomination "ARLACEL 83" par la Société ICI | | 4,5 g |
| - Mélange de mono- et distérate de glycérol et de stéarate de potassium | | 1 g |
| - Composé de formule ci-dessous préparé selon le brevet français n° 2 281 744 | | 20 g |

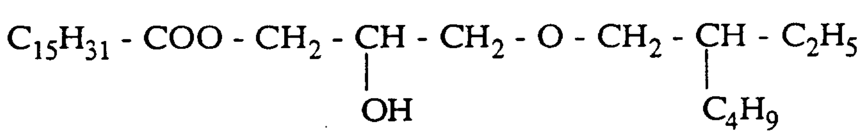

| | | |
|---|---|---|
| - Huile de vaseline | | 7 g |
| - Parfum, conservateurs | qs | |
| - Eau | qsp | 100 g |

## Revendications

1. Composition cosmétique filtrant le rayonnement ultraviolet de longueurs d'onde comprises entre 280 et 400nm, caractérisée par le fait qu'elle comprend, dans un support cosmétiquement acceptable, au moins un polymère hydrocarboné liposoluble synthétique ou naturel porteur d'au moins un groupement absorbant les ultraviolets et au moins un organopolysiloxane porteur d'au moins un groupe absorbant les ultraviolets, le rapport en poids du polymère filtre à l'organopolysiloxane filtre étant compris entre 0,1 et 5.

2. Composition cosmétique filtrante selon la revendication 1, caractérisée par le fait que le rapport en poids du polymère filtre à l'organopolysiloxane filtre est compris entre 0,2 et 2,5.

3. Composition cosmétique selon la revendication 1 ou 2, caractérisée par le fait que les polymères filtres hydrocarbonés liposolubles sont des polymères à structure polyéthylénique éventuellement substituée, de polyéthylèneimine, de chitine et de chitosane, sur lesquels sont greffées des molécules absorbant le rayonnement ultraviolet par l'intermédiaire d'une fonction ester, amide, éther, thioéther, sulfonyle ou acyle.

4. Composition cosmétique selon la revendication 1 ou 2, caractérisée par le fait que les polymères filtres hydrocarbonés liposolubles sont des polymères résultant de l'homo- ou de la copolymérisation de molécules absorbant le rayonnement ultraviolet portant un groupement insaturé choisi parmi les radicaux allyle, vinyle, acrylamide, méthacrylamide, vinyloxycarbonylméthyle, acrylamidoalkyle et notamment acrylamidométhyle, méthacrylamidoalkyle, acrylamido(phényl)alkyle, méthacrylamido(phényl)-alkyle, acryloxy, acryloxyalkyle et acryloxypolyoxyéthylène.

5. Composition cosmétique selon la revendication 4, caractérisée par le fait que les polymères filtres hydrocarbonés liposolubles résultent de l'homo- ou de la copolymérisation de molécules absorbant le rayonnement ultraviolet portant un groupement insaturé avec d'autres monomères insaturés choisis parmi les acides acrylique, méthacrylique, itaconique, crotonique ou leurs esters, l'acrylamide et ses dérivés, le méthacrylamide et ses dérivés, l'acrylonitrile, le méthacrylonitrile, le styrène, l'$\alpha$-méthylstyrène, l'isoprène, le butadiène, l'éthylène, le propylène, les esters vinyliques, les chlorures et fluorures de vinyle, le chlorure de vinylidène, la N-vinylpyrrolidone, la N-méthacryloyl D-glucosamine et les monoesters et diesters des acides maléique et fumarique.

6. Composition cosmétique selon l'une quelconque des revendications 3 à 5, caractérisée par le fait que les molécules absorbant le rayonnement ultraviolet sont choisies parmi le benzylidènecamphre, l'isophtalylidènecamphre et le téréphtalylidène camphre éventuellement substitués sur le noyau benzénique, l'acide cinnamique éventuellement substitué par un ou plusieurs groupes alcoxy inférieurs et ses esters, l'acide salicylique et ses esters, l'acide benzoïque et ses esters, l'acide p-aminobenzoïque et ses dérivés alkylés sur le groupement amino et leurs esters, les hydroxybenzophénones éventuellement substituées, le dibenzoylméthane éventuellement substitué, le benzotriazole et les 2-arylbenzotriazoles, les 2-arylbenzimidazoles, les 2-arylbenzofurannes, les 2-arylbenzoxazoles, les 2-arylindoles, les mono- ou diphénylcyanoacrylates et les absorbeurs de structure coumarinique.

7. Composition cosmétique selon l'une quelconque des revendications 1 à 6, caractérisée par le fait qu'elle contient un organo polysiloxane ou silicone filtre comportant dans sa molécule au moins une unité de formule :

$$X-\overset{R'_a}{\overset{|}{Si}}-O_{\frac{3-a}{2}} \qquad (VI)$$

dans laquelle R' désigne un groupe hydrocarboné saturé ou insaturé en $C_1$-$C_{30}$, un groupe hydrocarboné halogéné en $C_1$-$C_8$ ou un groupe triméthylsilyloxy,

a = 1 ou 2;
X = -A - Y

où A représente un radical divalent hydrocarboné aliphatique ou aromatique comportant au moins 2 atomes de carbone et renfermant éventuellement un ou plusieurs atomes d'oxygène; Y représente le reste d'une molécule filtrant le rayonnement ultraviolet.

**8.** Composition cosmétique selon la revendication 7, caractérisée par le fait que le diorganopolysiloxane comporte en outre des unités ayant pour formules :

$$R'_b - Si\,O_{\frac{4-b}{2}} \quad (VII) \quad \text{et} \quad Z - \overset{R'_a}{\overset{|}{Si}} - O_{\frac{3-a}{2}} \quad (VIII)$$

dans lesquelles R' et a ont les significations indiquées dans la revendication 7; b est un nombre entier égal à 1, 2 ou 3; Z = -O-Y, Y ayant la même signification que dans la revendication 7, au moins 40% en nombre des radicaux R' désignant méthyle.

**9.** Composition cosmétique selon la revendication 7 ou 8, caractérisée par le fait que le reste Y d'une molécule filtrant le rayonnement ultraviolet est un reste de benzylidène camphre non substitué ou substitué sur le noyau benzénique par des radicaux hydroxy, alkyle ou alcoxy en $C_1$-$C_6$; benzalmalonate de dialkyle en $C_1$-$C_8$, non substitué ou substitué sur le noyau benzénique par des radicaux hydroxy, alkyle ou alcoxy en $C_1$-$C_6$; 2-(2'-hydroxyphényl)benzotriazole non substitué ou portant sur l'un des noyaux aromatiques des substituants alkyle en $C_1$-$C_8$, alcényle en $C_2$-$C_8$, halogène, alcoxy, carboxy, hydroxy ou amino; dibenzoylméthane non substitué ou portant des substituants alkyle ou alcoxy en $C_1$-$C_8$ ou hydroxy; benzophénone non substitué ou portant des substituants alkyle ou alcoxy en $C_1$-$C_8$ ou hydroxy; benzoate substitué par des radicaux hydroxy, alcoxy en $C_1$-$C_6$, amino ou mono ou di($C_1$-$C_6$ alkyl)amino; cinnamate non substitué ou portant des substituants hydroxy, alkyle ou alcoxy en $C_1$-$C_6$, amino ou mono ou di($C_1$-$C_6$) alkylamino; bis- ou tris (phénylacrylate) non substitué ou substitué par des radicaux hydroxy ou alcoxy en $C_1$-$C_4$.

**10.** Composition cosmétique filtrante selon l'une quelconque des revendications 1 à 9, caractérisée par le fait qu'elle constitue une composition protectrice de l'épiderme humain sous forme d'huile, de dispersion vésiculaire, d'émulsion, de lotion, de gel, de bâtonnet solide ou sous forme de composition pour aérosol et contient 0,5 à 15% en poids de polymère filtre et silicone filtre par rapport au poids total de la composition.

**11.** Composition cosmétique selon l'une quelconque des revendications 1 à 9, caractérisée par le fait qu'elle constitue une composition antisolaire sous forme d'émulsion, de lotion, d'huile, de dispersion vésiculaire, de gel, de bâtonnet solide ou sous forme de composition pour aérosol, et qu'elle contient outre le polymère filtre et la silicone filtre, d'autres filtres UV-B ou UV-A, la concentration totale en agents filtrants étout comprise eutre 0,5 et 20% en poids sur la base du poids total de la composition.

**12.** Composition cosmétique selon l'une quelconque des revendications 1 à 9, utilisée pour la protection des cheveux contre le rayonnement ultraviolet, caractérisée par le fait qu'elle se présente sous forme de shampooing, de lotion, de gel ou émulsion à rincer, avant ou après shampooing, avant ou après coloration et décoloration, avant ou après permanente ou défrisage, sous forme de lotion ou gel coiffant ou traitant, de lotion ou gel pour le brushing ou la mise en plis, de laque pour cheveux, de composition de permanente ou de défrisage, de coloration ou décoloration des cheveux et contient 0,25 à 5% en poids par rapport au poids total de la composition d'au moins un polymère filtre et d'au moins une silicone filtre.

**13.** Composition cosmétique filtrante selon l'une quelconque des revendications 1 à 12, caractérisée par le fait qu'elle comprend un poly(4'-acrylamidométhyl benzylidène camphre) et un polydiméthylsiloxane à

greffons 4'-triméthylèneoxy-3-benzylidène camphre.

**14.** Composition cosmétique selon l'une quelconque des revendications 1 à 12, caractérisée par le fait qu'elle contient un poly(3'-acrylamidométhyl-4'-hexyloxybenzylidène camphre) et un polydiméthylsiloxane à greffons 2-(3'-triméthylène-2'-hydroxy-5'-méthyl)phénylbenzotriazole.

**15.** Composition cosmétique filtrante selon l'une quelconque des revendications 1 à 12, caractérisée par le fait qu'elle contient un polymère filtre comportant des motifs 2-(3'-acrylamidométhyl-2'-hydroxy-5'-tert.-octylphényl)benzotriazole et un polydiméthylsiloxane à greffons 2-méthyl 3-(4'-diméthylamino benzoyloxy) propylène.

**16.** Composition cosmétique filtrante selon l'une quelconque des revendications 1 à 12, caractérisée par le fait qu'elle contient un poly (4'-acrylamidométhyl benzylidène camphre) et un mélange de poly diméthylsiloxanes à greffons 4'-triméthylèneoxy-3-benzylidène camphre et 4'-oxy-3-benzylidène camphre.

**17.** Composition cosmétique filtrante selon l'une quelconque des revendications 1 à 12, caractérisée par le fait qu'elle contient un polymère filtre comportant des motifs 2-(3'-acrylamidométhyl-2'-hydroxy-5'-tert.-octylphényl)benzotriazole et un polydiméthylsiloxane à greffons 2-(3'-triméthylène-2'-hydroxy-5'-méthylphényl)benzotriazole.

**18.** Procédé cosmétique de protection de l'épiderme humain et des cheveux contre le rayonnement ultraviolet des longueurs d'onde comprises entre 280 et 400 nm, caractérisé par le fait que qu'il consiste à appliquer sur la peau ou les cheveux une quantité efficace d'une composition cosmétique selon l'une quelconque des revendications 1 à 17.

## Claims

**1.** Cosmetic composition screening out ultraviolet radiation of wavelengths between 280 and 400 nm, characterized in that it comprises, in a cosmetically acceptable vehicle, at least one synthetic or natural fat-soluble carbon/hydrogen-based polymer bearing at least one ultraviolet-absorbing group, and at least one organopolysiloxane bearing at least one ultraviolet-absorbing group, the weight ratio of the screening polymer to the screening organopolysiloxane being between 0.1 and 5.

**2.** Screening cosmetic composition according to Claim 1, characterized in that the weight ratio of the screening polymer to the screening organopolysiloxane is between 0.2 and 2.5.

**3.** Cosmetic composition according to Claim 1 or 2, characterized in that the fat-soluble carbon/hydrogen-based screening polymers are polymers of polyethylenimine, of chitin and of chitosan having an optionally substituted polyethylene structure, onto which polymers ultraviolet radiation-absorbing molecules are grafted via an ester, amide, ether, thioether, sulphonyl or acyl function.

**4.** Cosmetic composition according to Claim 1 or 2, characterized in that the fat-soluble carbon/hydrogen-based screening polymers are polymers resulting from the homo- or copolymerization of ultraviolet radiation-absorbing molecules bearing an unsaturated group chosen from allyl, vinyl, acrylamide, methacrylamide, vinyloxycarbonylmethyl, acrylamidoalkyl, and in particular acrylamidomethyl, methacrylamidoalkyl, acrylamido(phenyl)alkyl, methacrylamido(phenyl)alkyl, acryloxy, acryloxyalkyl and acryloxypolyethylene radicals.

**5.** Cosmetic composition according to Claim 4, characterized in that the fat-soluble carbon/hydrogen-based screening polymers result from the homo- or copolymerization of ultraviolet radiation-absorbing molecules bearing an unsaturated group with other unsaturated monomers chosen from acrylic, methacrylic, itaconic and crotonic acids or their esters, acrylamide and its derivatives, methacrylamide and its derivatives, acrylonitrile, methacrylonitrile, styrene, $\alpha$-methylstyrene, isoprene, butadiene, ethylene, propylene, vinyl esters, vinyl chlorides and fluorides, vinylidene chloride, N-vinylpyrrolidone, N-methacryloyl-D-glucosamine and the monoesters and diesters of maleic and fumaric acids.

6. Cosmetic composition according to any one of Claims 3 to 5, characterized in that the ultraviolet radiation-absorbing molecules are chosen from benzylidenecamphor, isophtalylidenecamphor and terephtalylidenecamphor, optionally substituted on the benzene ring, cinnamic acid optionally substituted with one or more lower alkoxy groups and its esters, salicylic acid and its esters, benzoic acid and its esters, p-aminobenzoic acid and its derivatives alkylated on the amino group and their esters, optionally substituted hydroxybenzophenones, optionally substituted dibenzoylmethane, benzotriazole and 2-arylbenzotriazoles, 2-arylbenzimidazoles, 2-arylbenzofurans, 2-arylbenzoxazoles, 2-arylindoles, mono- or diphenylcyanoacrylates and absorbers with a coumarin structure.

7. Cosmetic composition according to any one of Claims 1 to 6, characterized in that it contains a screening organopolysiloxane or silicone containing in its molecule at least one unit of formula:

$$X-\overset{R'_a}{\overset{|}{Si}}-O_{\frac{3-a}{2}} \quad (VI)$$

in which R' denotes a saturated or unsaturated $C_1$-$C_{30}$ carbon/hydrogen-based group, a halogenated $C_1$-$C_8$ carbon/hydrogen-based group or a trimethylsilyloxy group,

a = 1 or 2;
X = -A - Y

where A represents a bivalent aliphatic or aromatic carbon/hydrogen-based radical containing at least 2 carbon atoms and optionally including one or more oxygen atoms; Y represents the residue of a molecule screening out ultraviolet radiation.

8. Cosmetic composition according to Claim 7, characterized in that the diorganopolysiloxane contains, in addition, units having the formulae:

$$R'_b-Si\,O_{\frac{4-b}{2}} \quad (VII) \quad \text{and} \quad Z-\overset{R'_a}{\overset{|}{Si}}-O_{\frac{3-a}{2}} \quad (VIII)$$

in which R' and a have the meanings stated in Claim 7; b is an integer equal to 1, 2 or 3; Z = -O-Y, Y having the same meaning as in Claim 7, at least 40% in numerical terms of the radicals R' denoting methyl.

9. Cosmetic composition according to Claim 7 or 8, characterized in that the residue Y of a molecule screening out ultraviolet radiation is a benzylidenecamphor residue unsubstituted or substituted on the benzene ring with hydroxyl or $C_1$-$C_6$ alkyl or alkoxy radicals; a $C_1$-$C_8$ dialkylbenzalmalonate residue unsubstituted or substituted on the benzene ring with hydroxyl or $C_1$-$C_6$ alkyl or alkoxy radicals; a 2-(2'-hydroxyphenyl)benzotriazole residue unsubstituted or bearing $C_1$-$C_8$ alkyl, $C_2$-$C_8$ alkenyl, halogen, alkoxy, carboxyl, hydroxyl or amino substituents on one of the aromatic rings; a dibenzoylmethane residue unsubstituted or bearing $C_1$-$C_8$ alkyl or alkoxy or hydroxyl substituents; a benzophenone residue unsubstituted or bearing $C_1$-$C_8$ alkyl or alkoxy or hydroxyl substituents; a benzoate residue substituted with hydroxyl, $C_1$-$C_6$ alkoxy, amino or mono- or di($C_1$-$C_6$ alkyl)amino radicals; a cinnamate residue unsubstituted or bearing hydroxyl, $C_1$-$C_6$ alkyl or alkoxy, amino or mono- or di($C_1$-$C_6$ alkyl)-amino substituents; or a bis- or tris(phenylacrylate) residue unsubstituted or substituted with hydroxyl or $C_1$-$C_4$ alkoxy radicals.

10. Screening cosmetic composition according to any one of Claims 1 to 9, characterized in that it constitutes a composition for protecting the human epidermis in the form of an oil, vesicular dispersion, emulsion, lotion, gel or solid stick or in the form of a composition for dispensing in aerosol form, and

contains 0.5 to 15% by weight of screening polymer and screening silicon relative to the total weight of the composition.

**11.** Cosmetic composition according to any one of Claims 1 to 9, characterized in that it constitutes an antisun composition in the form of an emulsion, lotion, oil, vesicular dispersion, gel or solid stick or in the form of a composition for dispensing in aerosol form, and in that it contains, besides the screening polymer and the screening silicone, other UV-B or UV-A screening agents, the total composition of screening agents being between 0.5 and 20% by weight based on the total weight of the composition.

**12.** Cosmetic composition according to any one of Claims 1 to 9, used for the protection of hair against ultraviolet radiation, characterized in that it takes the form of a shampoo, lotion, gel or emulsion to be rinsed, before or after shampooing, before or after dyeing and bleaching, before or after permanent-waving or straightening, or the form of a styling or treatment lotion or gel, lotion or gel for blow drying or setting, hair lacquer or composition for permanent-waving or straightening, dyeing or bleaching hair, and contains 0.25 to 5% by weight relative to the total weight of the composition of at least one screening polymer and of at least one screening silicone.

**13.** Screening cosmetic composition according to any one of Claims 1 to 12, characterized in that it comprises a poly[4'-(acrylamidomethyl)benzylidene camphor] and a polydimethyl siloxane containing 4'-trimethyleneoxy-3-benzylidenecamphor grafts.

**14.** Cosmetic composition according to any one of Claims 1 to 12, characterized in that it contains a poly-(3'-acrylamidomethyl-4'-hexyloxybenzylidenecamphor) and a polydimethylsiloxane containing 2-(3'-trimethylene-2'-hydroxy-5'-methyl)phenylbenzotriazole grafts.

**15.** Screening cosmetic composition according to any one of Claims 1 to 12, characterized in that it contains a screening polymer containing 2-(3'-acrylamidomethyl-2'-hydroxy-5'-tert-octylphenyl)-benzotriazole units and a polydimethylsiloxane containing 2-methyl-3-(4'-dimethylaminobenzoyloxy)-propylene grafts.

**16.** Screening cosmetic composition according to any one of Claims 1 to 12, characterized in that it contains a poly[4'-(acrylamidomethyl)benzylidenecamphor] and a mixture of polydimethylsiloxanes containing 4'-trimethyleneoxy-3-benzylidene camphor and 4'-oxy-3-benzylidenecamphor grafts.

**17.** Screening cosmetic composition according to any one of Claims 1 to 12, characterized in that it contains a screening polymer containing 2-(3'-acrylamidomethyl-2'-hydroxy-5'-tert-octylphenyl)-benzotriazole units and a polydimethylsiloxane containing 2-(3'-trimethylene-2'-hydroxy-5'-methylphenyl)benzotriazole grafts.

**18.** Cosmetic process for protecting the human epidermis and hair against ultraviolet radiation of wavelengths between 280 and 400 nm, characterized in that it consists in applying to the skin or hair an effective amount of a cosmetic composition according to any one of Claims 1 to 17.

## Patentansprüche

**1.** Kosmetische Zusammensetzung, die die ultraviolette Strahlung von Wellenlängen von 280 bis 400 nm filtert,
dadurch **gekennzeichnet**, daß
sie, in einem kosmetisch geeigneten Trägermedium, mindestens ein fettlösliches synthetisches oder natürliches Kohlenwasserstoffpolymer mit mindestens einer die UV-Strahlen absorbierenden Gruppe und mindestens ein Organopolysiloxan mit mindestens einer die UV-Strahlen absorbierenden Gruppe enthält, wobei das Gewichtsverhältnis des Polymer-Filterstoffs zum Organopolysiloxan-Filterstoff 0,1 bis 5 beträgt.

**2.** Kosmetische Filter-Zusammensetzung gemäß Anspruch 1,
dadurch **gekennzeichnet**, daß
das Gewichtsverhältnis des Polymer-Filterstoffs zum Organopolysiloxan-Filterstoff 0,2 bis 2,5 beträgt.

**3.** Kosmetische Zusammensetzung gemäß Anspruch 1 oder 2,
dadurch **gekennzeichnet**, daß
die fettlöslichen Kohlenwasserstoff-Polymerfilterstoffe Polymere mit gegebenenfalls substituierter polyethylenischer Struktur von Polyethylenimin, Chitin und Chitosan sind, auf welche die ultraviolette Strahlung absorbierende Moleküle über die Zwischenproduktverbindung einer Ester-, Amid-, Ether-, Thioether-, Sulfonyl- oder Acyl-Funktion gepfropft sind.

**4.** Kosmetische Zusammensetzung gemäß Anpruch 1 oder 2,
dadurch **gekennzeichnet**, daß
die fettlöslichen Kohlenwasserstoffpolymer-Filterstoffe Polymere sind, die durch Homo- oder Copolymerisation von die ultraviolette Strahlung absorbierenden Molekülen hergestellt sind, welche eine ungesättigte Gruppe aufweisen, die aus Allyl-, Vinyl-, Acrylamid-, Methacrylamid, Vinyoxycarbonylmethyl-, Acrylamidoalkyl- und insbesondere aus Acrylamidomethyl-, aus Methacrylamidoalkyl-, Acrylamido(phenyl)alkyl-, Methacrylamido(phenyl)alkyl-, Acryloxy-, Acryloxyalkyl- und Acryloxypolyoxethylen-Resten ausgewählt ist.

**5.** Kosmetische Zusammensetzung gemäß Anspruch 4,
dadurch **gekennzeichnet**, daß
die fettlöslichen Kohlenwasserstoffpolymer-Filterstoffe durch Homo- oder Copolmerisation von die ultraviolette Strahlung absorbierenden Molekülen, die eine ungesättigte Gruppe aufweisen, mit weiteren ungesättigten Monomeren hergestellt sind, die aus Acryl-, Methacryl-, Itacon-, Crotonsäuren oder deren Estern, aus Acrylamid und seinen Derivaten, Methacrylamid und seinen Derivaten, Acrylnitril, Methacrylnitril, Styrol,
$\alpha$-Methylstyrol, Isopren, Butadien, Ethylen, Propylen, Vinylestern, Vinylchloriden und -fluoriden, Vinylidenchlorid, N-Vinylpyrrolidon, N-Methacryloyl-D-glucosamin sowie aus Monoestern und Diestern von Malein- und Fumarsäuren ausgewählt sind.

**6.** Kosmetische Zusammenstzung gemäß einem jeden der Ansprüche 3 bis 5,
dadurch **gekennzeichnet,** daß
die die ultraviolette Strahlung absorbierenden Moleküle aus gegebenenfalls am Benzolkern substituierten Benzylidenkampfer-, Isophthalylidenkampfer- und Terephthalylidenkampfer-Resten, aus gegebenenfalls mit einer oder mehreren Niedrigalkoxygruppen substituierten Zimtsäure-Resten und deren Estern, aus Salicylsäure und ihren Estern, Benzoesäure und ihren Estern, p-Aminobenzoesäure und ihren an der Aminogruppe alkylierten Derivaten sowie deren Estern, aus gegebenenfalls substituierten Hydroxybenzophenon-Resten, aus gegebenenfalls substituierten Dibenzoylmethan-, Benztriazol- und 2-Arylbenztriazol-, 2-Arylbenztriazol-, 2-Arylbenzofuran-2-Arylbenzoxazol-, 2-Arylindol-, Mono- oder Diphenylcyanacrylat-Resten sowie aus Absorberresten mit Coumarinstruktur ausgewählt sind.

**7.** Kosmetische Zusammensetzung gemäß einem jeden der Ansprüche 1 bis 6,
dadurch **gekennzeichnet**, daß
sie einen Organopolysiloxan- oder Silikon-Filterstoff enthält, der in seinem Molekül mindestens eine Einheit der Formel aufweist:

$$X - \overset{\overset{\displaystyle R'_a}{|}}{Si} - O_{\frac{3-a}{2}} \qquad (VI)$$

worin R' eine gesättigte oder ungesättigte $C_{1-30}$-Kohlenwasserstoff-, halogenierte $C_{1-8}$-Kohlenwasserstoff- oder eine Trimethylsilyloxygruppe darstellt,

a = 1 oder 2,
X = -A-Y,

worin A einen aliphatischen oder aromatischen zweiwertigen Kohlenwasserstoffrest mit mindestens zwei Kohlenstoffatomen und gegebenenfalls einem oder mehreren Sauerstoffatomen und Y den Rest eines die ultraviolette Strahlung filternden Moleküls darstellen.

**8.** Kosmetische Zusammensetzung gemäß Anspruch 7,
dadurch **gekennzeichnet**, daß
das Diorganopolysiloxan ausserdem Einheiten mit den Formeln aufweist:

$$R'_b - Si\,O_{\frac{4-b}{2}} \quad (VII) \qquad \text{und} \qquad Z - \underset{}{\overset{R'_a}{\overset{|}{Si}}} - O_{\frac{3-a}{2}} \quad (VIII)$$

in denen R' und a die in Anspruch 7 angegebenen Bedeutungen haben, b eine ganze Zahl gleich 1, 2 oder 3 ist und Z -O-Y ist, wobei Y dieselbe Bedeutung wie in Anspruch 7 hat, und wobei mindestens 40% der Anzahl der Reste R' den Methylrest bedeuten.

**9.** Kosmetische Zusammensetzung gemäß Anspruch 7 oder 8,
dadurch **gekennzeichnet**, daß
der Rest Y des die ultraviolette Strahlung filternden Moleküls ein Benzylidenkampfer-Rest, der nicht substituiert oder am Benzolkern mit Hydroxy-, $C_{1-6}$-Alkyl- oder - Alkoxyresten substituiert ist, ein $C_{1-8}$-Dialkylbenzalmalonat-Rest,der nicht substituiert oder am Benzolkern mit Hydroxy-, $C_{1-6}$-Alkyl- oder -Alkoxyresten substituiert ist, ein 2-(2'-Hydroxyphenyl)benztriazol-Rest, der nicht substituiert ist oder an einem der aromatischen Kerne $C_{1-8}$-Alkyl-, $C_{2-8}$-Alkenyl-, Halogen-, Alkoxy-, Carboxy-, Hydroxy- oder Amino-Substituenten aufweist, ein Dibenzoylmethan-Rest, der nicht substituiert ist oder $C_{1-8}$-Akyl- oder -Alkoxy- oder Hydroxy-Substituenten aufweist, ein Benzophenon-Rest, der nicht substituiert ist oder $C_{1-8}$-Alkyl oder -Alkoxy- oder Hydroxy-Substituenten aufweist, ein Benzoat-Rest, der mit Hydroxy-, $C_{1-6}$-Alkoxy-, Amino- oder Mono- oder Di($C_{1-6}$)alkylaminoresten substituiert ist, ein Zimtsäurerest, der nicht substituiert ist oder Hydroxy-, $C_{1-6}$-Alkyl- oder -Alkoxy-, Amino- oder Mono- oder Di($C_{1-6}$)-alkylamino-Substituenten aufweist, oder ein Bis- oder Tris(phenylacrylat)-Rest ist, der nicht substituiert oder mit Hydroxy- oder $C_{1-4}$-Alkoxyresten substituiert ist.

**10.** Kosmetische Filter-Zusammensetzung gemäß einem jeden der Ansprüche 1 bis 9,
dadurch **gekennzeichnet**, daß
sie eine Zusammensetzung zum Schutz der menschlichen Haut in Form eines Öls, einer bläschenartigen Dispersion, einer Emulsion, Lotion, eines Gels, Feststoffstäbchens oder in Form einer Zusammensetzung für ein Aerosol darstellt und 0,5 bis 15 Gew.% Polymer-Filterstoff und Silikon-Filterstoff enthält, bezogen auf das Gesamtgewicht der Zusammensetzung.

**11.** Kosmetische Zusammensetzung gemäß einem jeden der Ansprüche 1 bis 9,
dadurch **gekennzeichnet**, daß
sie eine Sonnenschutzmittelzusammensetzung in Form einer Emulsion, Lotion, eines Öls, einer bläschenartigen Dispersion, eines Gels, Feststoffstäbchens oder in Form einer Zusammensetzung für ein Aerosol darstellt und zusätzlich zum Polymer-Filterstoff und Silikon-Filterstoff weitere UV-B- oder UV-A-Filterstoffe enthält, wobei die Gesamtkonzentration an Filtermitteln 0,5 bis 20 Gew.% beträgt, bezogen auf das Gesamtgewicht der Zusammensetzung.

**12.** Kosmetische Zusammensetzung gemäß einem jeden der Ansprüche 1 bis 9, zur Verwendung zum Schutz der Haare vor ultravioletter Strahlung,
dadurch **gekennzeichnet**, daß
sie in Form eines Shampoo, einer Lotion, eines Gels oder einer Emulsion zur Spülung, vor oder nach dem Schamponieren, vor oder nach einer Färbung oder Entfärbung, vor oder nach einer Dauerwelle oder einem Entfrisiervorgang, in Form einer Lotion oder eines Gels zum Frisieren oder Behandeln, einer Lotion oder eines Gels zum Bürsten oder zur Wellengebung, eines Haarlacks, einer Zusammensetzung zur Dauerwelle oder zum Entfrisieren, zur Färbung oder Entfärbung der Haare vorliegt und 0,25 bis 5 Gew.%, bezogen auf das Gesamtgewicht der Zusammensetzung, mindestens eines Polymer-Filterstoffs und mindestens eines Silikon-Filterstoffs enthält.

**13.** Kosmetische Filterzusammensetzung gemäß einem jeden der Ansprüche 1 bis 12, dadurch **gekennzeichnet**, daß sie einen Poly(4'-acrylamidomethylbenzylidenkampfer) und ein Polydimethylsiloxan mit gepfropften 4'-Trimethylenoxy-3-benzylidenkampfer-Resten enthält.

**14.** Kosmetische Zusammensetzung gemäß einem jeden der Ansprüche 1 bis 12, dadurch **gekennzeichnet**, daß sie einen Poly(3'-acrylamidomethyl-4'-hexyloxybenzylidenkampfer) und ein Polydimethylsiloxan mit gepfropften 2-(3'-Trimethylen-2'-hydroxy-5'-methyl)phenylbenztriazolresten enthält.

**15.** Kosmetische Filterzusammensetzung gemäß einem jeden der Ansprüche 1 bis 12, dadurch **gekennzeichnet,** daß sie einen Polymer-Filterstoff mit 2-(3'-Acrylamidomethyl-2'-hydroxy-5'-t-octylphenyl)benztriazol-Einheiten und ein Polydimethylsiloxan mit 2-Methyl-3-(4'-dimethylaminobenzoyloxy)propylen-Pfropfresten enthält.

**16.** Kosmetische Filterzusammensetzung gemäß einem jeden der Ansprüche 1 bis 12, dadurch **gekennzeichnet** daß sie einen Poly(4'-acrylamidomethylbenzylidenkampfer) und eine Mischung aus Polydimethylsiloxanen mit gepfropften 4'-Trimethylenoxy-3-benzylidenkamper- und 4'-Oxy-3-benzylidenkampfer-Resten enthält.

**17.** Kosmetische Filterzusammensetzung gemäß einem jeden der Ansprüche 1 bis 12, dadurch **gekennzeichnet,** daß sie einen Polymer-Filterstoff mit 2-(3'-Acrylamidomethyl-2'-hydroxy-5'-t-octylphenyl)benztriazol-Einheiten und ein Polysiloxan mit 2-(3'-Trimethylen-2'-hydroxy-5'-methylphenyl)benztriazol-Pfropfresten enthält.

**18.** Kosmetisches Verfahren zum Schutz der menschlichen Haut und der Haare vor ultravioletter Strahlung von Wellenlängen von 280 bis 400 nm, dadurch **gekennzeichnet,** daß man auf die Haut oder die Haare eine wirksame Menge einer kosmetischen Zusammensetzung gemäß einem jeden der Ansprüche 1 bis 17 aufträgt.